(19) Europäisches Patentamt
European Patent Office
Office européen des brevets

(11) Numéro de publication: **0 210 885**
**B1**

(12) **FASCICULE DE BREVET EUROPEEN**

(45) Date de publication du fascicule du brevet: **11.07.90**

(21) Numéro de dépôt: **86401368.5**

(22) Date de dépôt: **20.06.86**

(51) Int. Cl.$^5$: **C 07 D 213/30,**
C 07 D 213/80,
C 07 D 233/60,
C 07 D 295/14,
C 07 C 43/174, C 07 C 69/63,
A 61 K 31/415, A 61 K 31/44,
A 61 K 31/495

(54) **Dérivés d'alcools primaires halogéno biphényles utiles en thérapeutique dans le traitement de l'athérosclérose.**

(30) Priorité: **08.07.85 FR 8510528**

(43) Date de publication de la demande:
**04.02.87 Bulletin 87/06**

(45) Mention de la délivrance du brevet:
**11.07.90 Bulletin 90/28**

(84) Etats contractants désignés:
**AT BE CH DE GB IT LI LU NL SE**

(56) Documents cités:
**FR-A-1 460 659**
**FR-A-2 232 307**
**FR-A-2 233 046**
**FR-A-2 476 072**
**FR-A-2 498 449**
**US-A-2 527 963**

**Le dossier contient des informations techniques présentées postérieurement au dépôt de la demande et ne figurant pas dans le présent fascicule.**

(73) Titulaire: **PIERRE FABRE MEDICAMENT**
**125, Rue de la Faisanderie**
**F-75116 Paris (FR)**

(72) Inventeur: **Cousse, Henri La Foun de los Nobios**
**Chemin de Lastinos**
**F-81100 Castres (FR)**
Inventeur: **Delhon, André**
**36, rue d'Hector Berlioz**
**F-81100 Castres (FR)**
Inventeur: **Rieu, Jean-Pierre La Vixère Haute**
**Avenue du Sidobre**
**F-81100 Castres (FR)**
Inventeur: **Mouzin, Gilbert**
**21, rue Sainte Foy**
**F-81100 Castres (FR)**

(74) Mandataire: **Doat, Jean-Pierre**
**PIERRE FABRE S.A. Service Propriété Industrielle 17, Avenue Jean Moulin**
**F-81106 Castres Cédex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 210 885 B1

**Description**

La présente invention a pour objet des dérivés d'alcools primaires halogéno biphényles possédant des propriétés hypolipémiante et hypocholestérolémiante, leur procédé de préparation et leur application en tant que médicaments utiles dans le traitement et la prévention des troubles de l'athérosclérose.

Dans les brevets FR 2.476.072 et FR 2.498.449 déposés par la demanderesse, il est fait état de dérivés de l'acide halogénobiphényl carboxylique utiles dans le traitement des troubles provoqués par l'athérosclérose.

Les composés découverts par la demanderesse présentent une différence structurale par la présence d'un groupe fonctionnel éther ou ester.

L'invention a pour objet des dérivés d'alcools halogéno biphényles de formule générale (I):

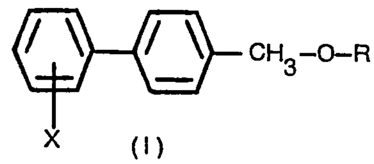

(I)

dans laquelle:

X représente un atome d'halogène et plus particulièrement le chlore, le brome ou le fluor en position ortho ou méta.

R représente un radical alcoyle, aminoalcoyle, pyridyl alcoyle ou acyle et plus particulièrement

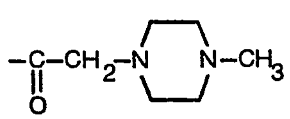
et
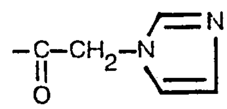
,

acétyle.

L'invention concerne également les sels des composés de formule I dans le cas où R est salifiable avec des acides minéraux ou organiques thérapeutiquement acceptables.

L'invention vise aussi un procédé de préparation des dérivés de formule I, par réaction des alcools primaires de formule II:

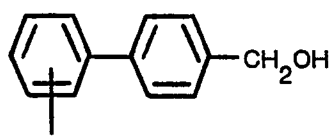

(II)

dans laquelle X a la même signification que dans la formule I avec composé de formule $R_nY$ dans laquelle R a la même signification que dans la formule I et Y représente un atome d'halogène (n=1) ou la fonction sulfate (n=2) selon le schéma réactionnel:

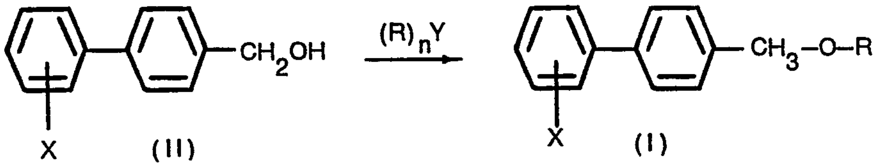


(II) (I)

La présente invention concerne de même l'utilisation des composés de formule I à titre de médicament, ainsi que les compositions pharmaceutiques contenant ces médicaments.

Les compositions pharmaceutiques selon la présente invention peuvent comporter un ou plusieurs composés de formule I éventuellement en association avec d'autres principes actifs.

Parmi les dérivés de formule I, on peut citer plus particulièrement les dérivés suivants:

Exemple 1

Hydrogénooxalate de (pyridyl-3 méthoxy méthyl)-4 chloro-2' biphényle (F 2832)

Un mélange de (chloro-2' biphényl)-4 méthanol (7 g, 32 mmoles) et de tétrabutylammonium hydrogéno sulfate (2,17 g, 64 mmoles) est agité à température ordinaire dans une solution de lessive de soude à 50% (25,6 g). On ajoute par portions le chlorhydrate de chlorure de nicotinyle (15,75 g, 96 mmoles), puis on poursuit l'agitation pendant 6 heures. Le mélange, coulé dans l'eau glacée, est extrait à l'éther (2 × 250 ml), lavé à l'eau (3 × 200 ml) et à l'eau salée (20 ml) et séché sur sulfate de sodium.

Après concentration sous vide et filtration sur gel de silice (250 g élution: chloroforme — méthanol 95/

5), on isole 7,8 g de produit. La base obtenue, reprise dans l'isopropanol est salifiée par l'acide oxalique (2 g/100 ml isopropanol).

On isole avec un rendement de 50 à le dérivé de formule:

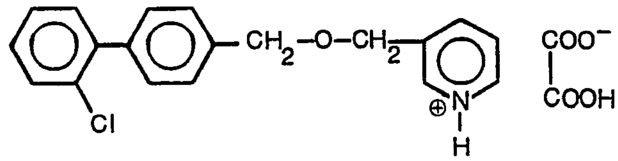

Formule brute: $C_{21}H_{18}Cl\ NO_5$
Masse moléculaire: 399.83
Point de fusion: 108°C
Chromatographie sur couche mince:
— support: gel de silice 60 F 254 Merck
— solvant: $CHCL_3$ — MeOH 95/5
— révélation: UV et iode
— Rf: 0.50
Solubilité: 0.1% dans l'eau.

D'une maniére analogue à celle décrite dans l'exemple 1, les composés suivants ont été préparés:

Exemple 2
(méthoxy méthyl)-4 chloro-2' biphényle (F 2816)

Le (chloro-2' biphényl)-4 méthanol traité par le sulfate de méthyle conduit au dérivé de formule:

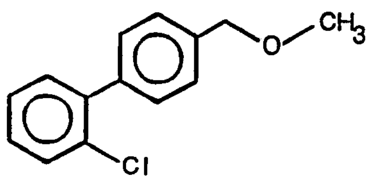

Formule brute: $C_{14}H_{14}Cl\ O$
Masse moléculaire: 232.71
Eb: 130°C/$10^{-3}$ mm — $n_D^{26}$: 1.5902
Chromatographie sur couche mince:
— support: gel de silice 60 F 254 Merck
— solvant: hexane — acétate d'éthyle 70/30
— révélation: UV
— Rf: 0.60
Solubilité: 1% dans le propylène glycol.

Exemple 3
(éthoxy méthyl)-4 chloro-2' biphényle (F 2815)

Selon le mode opératoire de l'exemple 1, en substituation le chlorure de nicotinyle par le bromure d'éthyle, on obtient le dérivé de structure suivante:

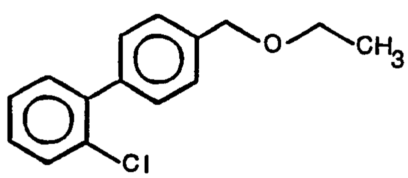

Formule brute: $C_{15}H_{15}Cl\ O$
Masse moléculaire: 246,74
Eb: ($10^{-3}$ mm) 160°C — $n_D^{25}$: 1.5760
Chromatographie sur couche mince:
— support: gel de silice 60 F 254 Merck
— solvant: hexane — acétate d'éthyle 70/30
— révélation: UV
— Rf: 0.61
Solubilité: 0.6% dans le propylène glycol.

Exemple 4

(n-butoxyméthyl)-4 chloro-2′ biphényle (F 2831)

Le traitement du (chloro-2′ biphényl)-4 méthanol par le bromure de butyle selon le mode opératoire de l'exemple 1 conduit au dérivé de structure:

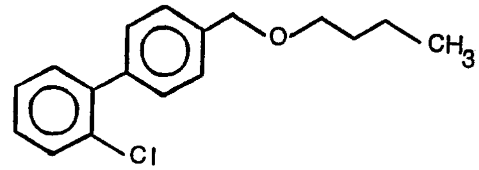

Formule brute: $C_{17}H_{19}Cl\ O$
Masse moléculaire: 274,79
Eb: ($10^{-3}$ mm) 160°C — $n_D^{22}$: 1.5640
Chromatographie sur couche mince:
— support: gel de silice 60 F 254 Merck
— solvant: hexane — acétate d'éthyle 70/30
— révélation: UV
— Rf: 0.70
Solubilité: 0.3% dans le propylène glycol.

Exemple 5

hydrogénooxalate de [(diméthylamino-2 éthoxy) méthyl]-4 chloro-2′ biphényle (F 2850)

Un mélange de (chloro-2′ biphényl)-4 méthanol (7 g, 30 mmoles) et de chlorhydrate de diéthylamino-2 éthylchlorure (13.05 g, 90 mmoles) est refroidi au bain de glace puis traité sous agitation par 16.1 ml de lessive de soude à 50% (0.3 mole). L'agitation est maintenue 24 h à température ambiante puis le mélange réactionnel est versé dans l'eau froide et extrait à l'éther (2 × 100 ml).

La phase ethérée est lavée à l'eau (3 × 100 ml) puis à l'eau salée (150 ml) séchée sur sulfate et évaporée sous vide. La phase obtenue (8.5 g), reprise dans l'acétate d'éthyle est salifiée par une solution d'acide oxalique dans l'acétate d'éthyle (2.65 g/100 ml). Les cristaux obtenus après filtration, essorage et séchage, sont recristallisés dans l'éther isopropylique (320 ml). On isole avec un rendement de 65% le composé:

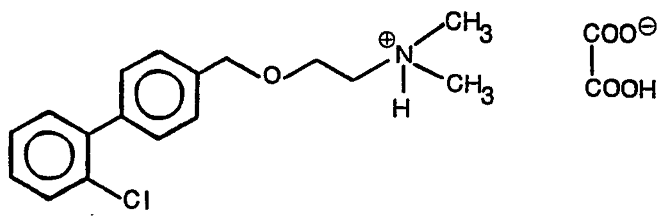

Formule brute: $C_{19}H_{22}Cl\ NO_5$
Masse moléculaire: 379.84
Point de fusion: 138.5°C
Chromatographie sur couche mince:
— support: gel de silice 60 F 254 Merck
— solvant: $CHCl_3$ — MeOH — $NH_4OH$
— révélateur: UV et iode
— Rf: 0.50
Solubilité: 0.25% dans l'eau.

Exemple 6

Dihydrogénomaléate de [(méthyl-4 (1-4) pipérazinyl-1) acétoxyméthyl]-4 chloro-2′ biphényle (F 2876)

1) Préparation du chloro-2′ (chloroacétoxyméthyl)-4 biphényle

Le (chloro-2′ biphényle)-4 méthanol (15 g, 68.5 mmoles) dans le DMF (150 ml) est traité goutte à goutte à température de 0°C par le chlorure de chloracétyle (8.5 g, 75.3 mmoles).

Après une heure d'agitation à température ambiante, la solution est coulée dans l'eau glacée, extraite à l'éther (200 ml), lavée à l'eau (6 × 200 ml) et séchée sur sulfate.

La solution est évaporée à sec sous vide (M = 20 g).

2) Condensation de l'amine

Une solution du dérivé précédent (10 g, 34 mmoles) dans 100 ml de DMF est refroidie à 0°C puis traitée par la monométhyl pipérazine (13.6 g, 136 mmoles). Le mélange est maintenu sous agitation à température ambiante pendant 1 h, puis coulée sur de l'eau glacée (400 ml). La base organique est extraite à l'éther (2 × 200 ml), lavée à l'eau (5 × 200 ml), au bicarbonate (100 ml) puis à l'eau salée (2 × 100 ml). On obtient après séchage sur sulfate et évaporation sous vide 10.8 g de produit.

La base, reprise dans l'alcool isopropylique (500 ml) est ajoutée à une solution d'acide maléique dans

l'alcool isopropylique (7 g/150 ml). Les cristaux obtenus, après essorage et séchage sont recristallisés dans le mélange isopropanol — éthanol 90/10.

On isole, avec un rendement de 60% le composé:

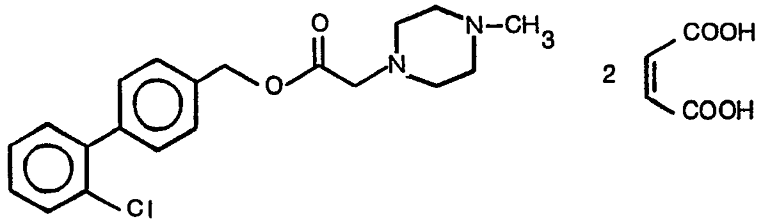

Formule brute: $C_{28}H_{31}Cl\ N_2\ O_{10}$
Masse moléculaire: 591.01
Point de fusion: 153°C
Chromatographie sur couche mince:
— support: gel de silice 60 F 254 Merck
— solvant: $CHCl_3$ — MeOH — $NH_4OH$ 90/9/1
— révélation: UV et iode
— Rf: 0.50
Solubilité: 0.25% dans l'eau.

Exemple 7

Hydrogénomaléate d'(imidazolyl acétoxy méthyl)-4 chloro-2' biphényle (F 2875)

Une solution de chloro-2' (chloroacétoxyméthyl)-4 biphenyle (10 g, 34 mmoles) obtenue selon l'exemple 6.1, en solution dans le DMF (100 ml) est traitée à 0°C par l'imidazole (9.25 g, 136 mmoles) puis la triéthyl amine (12 ml).

Le mélange est agité 24 h à température ambiante puis coulé sur de l'eau glacée. Après extraction à l'éther (2 × 250 ml), lavage à l'eau (5 × 200 ml) au bicarbonate (2 × 100 ml) puis à l'eau salée, la phase organique est séchée sur sulfate et concentrée sous vide (M = 8.75 g). La base reprise dans l'isopropanol (100 ml) est ajoutée à une solution d'acide maléique (3.1 g/100 ml isopropanol). Après cristallisation à froid, le produit est recristallisé dans l'isopropanol (100 ml).

On obtient avec un rendement de 63% le composé de formule:

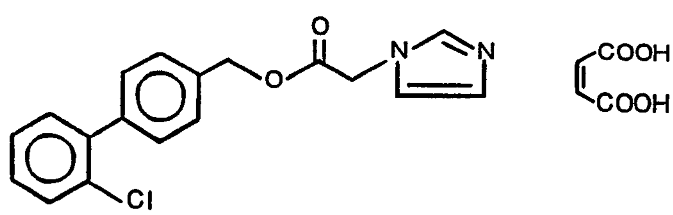

Formule brute: $C_{22}H_{19}ClN_2O_6$
Masse moléculaire: 442.855
Point de fusion: 103°C
Chromatographie sur couche mince:
— support: gel de silice 60 F 254 Merck
— solvant: $CHCl_3$ — MeOH — $NH_4OH$ 90/9/1
— révélation: UV et iode
— Rf: 0.60
Solubilité: 0.5% dans l'eau.

Exemple 8

(pyridyl-3 carboxyméthyl)-4 chloro-2' biphényle (F 2882)

Une solution d'acide nicotinique (16.9 g, 137 mmoles) dans la pyridine (120 ml) est traitée goutte à goutte par le chlorure de thionyle (16.3 g, 137 mmoles) à température ambiante. Aprés une nuit sous agitation, on ajoute le (chloro-2' biphényl)-4 méthanol et laisse 24 h supplémentaire sous agitation.

Après évaporation de la pyridine et reprise à l'eau, le pH est ramené à 6 et la solution extraite à l'éther (3 × 100 ml). La phase ethérée est lavée à l'eau (2 × 100 ml) à l'acide citrique à 10% (2 × 100 ml), à l'eau salée puis séchée sur sulfate et concentrée sous vide.

Le produit brut est repris par l'acide chlorhydrique N/10 (50 ml) et précipite par 2 volumes d'eau. Après

recristallisation dans le mélange cyclohexane — éther isopropylique 75/25, on obtient avec un rendement de 62% le composé:

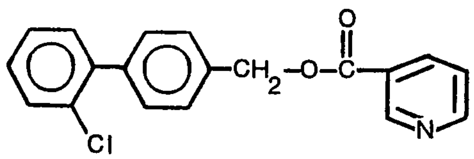

Formule brute: $C_{19}H_{14}ClNO_2$
Masse moléculaire: 323.78
Point de fusion: 65°C
Chromatographie sur couche mince:
— support: gel de silice 60 F 254 Merck
— solvant: hexane — acétate d'éthyle 50/50
— révélation: UV et iode
— Rf: 0.35
Solubilité: 0.5% dans le propylène glycol.

Exemple 9

acétoxy méthyl-4 chloro-2' biphényle (F 2855)

Une solution de (chloro-2' biphényl)-4 méthanol (6 g, 27 mmoles) et de chlorure d'acétyle (2.76 g, 35 mmoles) dans l'éther (70 ml) est traitée goutte à goutte à 0°C par la triéthylamine (4.35 g, 43 mmoles). Après 3 h d'agitation à température ambiante, la phase etherée est filtrée, lavée à la soude N, à l'eau puis à l'eau salée.

Après séchage sur sulfate, le filtrat est évaporé sous vide et l'huile résiduelle rectifiée sous vide pour donner avec un rendement de 92%, le composé:

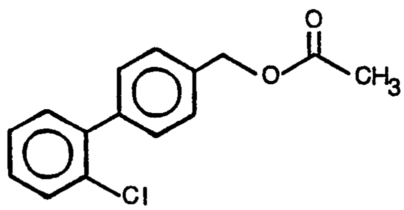

Formule brute: $C_{15}H_{13}ClO_2$
Masse moléculaire: 260.72
Eb ($4.10^{-3}$ mm Hg): 160°C — $n_D^{22}$: 1.5825
Chromatographie sur couche mince:
— support: gel de silice 60 F 254 Merck
— solvant: hexane — acétate d'éthyle 70/30
— révélation: UV et iode
— Rf: 0.60
Solubilité: 1% dans le propylène glycol.

Exemple 10

Hydrogénomaléate de chloro-2' (diéthylamino acétoxyméthyl)-4 biphényle (F 2870)

De façon similaire à l'exemple 7, l'intermédiaire chloro-2' (chloro acétoxyméthyl)-4 biphényle (9.1 g, 32 mmoles) traité par la diéthyl amine conduit au dérivé de formule suivante:

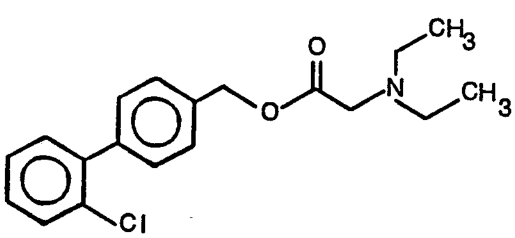

Formule brute: $C_{23}H_{26}Cl\ NO_6$
Masse moléculaire: 447.915
Point de fusion: 104°C
Chromatographie sur couche mince:
— support: gel de silice 60 F 254 Merck
— solvant: $CHCl_3$ — MeOH 95/5

— révélation: UV — iode
— Rf: 0.50
Solubilité: 0.33% dans l'eau.

Exemples 11 à 15

D'un manière similaire à celle décrite dans l'exemple 1, en utilisant les alcools et les dérivés halogénés correspondants, on prépare les composés suivants:

EXEMPLES FORMULE CHIMIQUE

Exemple 11: Chlorhydrate de (pyridyl-3 méthoxyméthyl)-4 fluoro-2' biphenyle

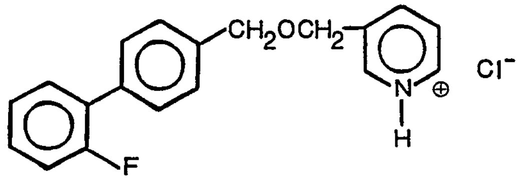

Exemple 12: Chlorhydrate de pyridyl-3 méthoxyméthyl)-4 chloro-3' biphényle

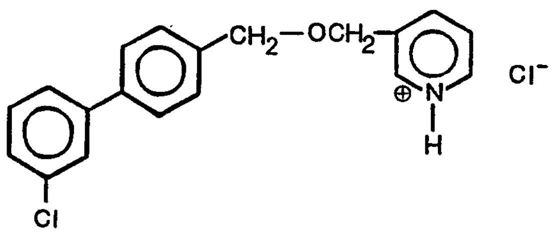

Exemple 13: (éthoxyméthyl)-4 chloro-3' biphényle

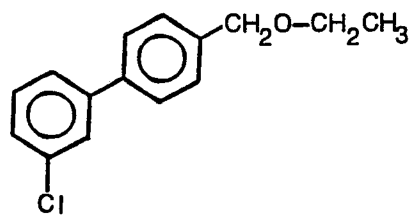

Exemple 14: (éthoxyméthyl)-4-bromo-3 biphényle

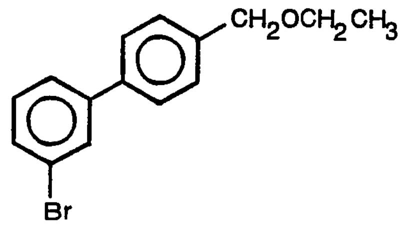

Exemple 15: Oxalate acide de (pyridyl-3 méthoxyméthyl)-4 bromo-2' biphényle

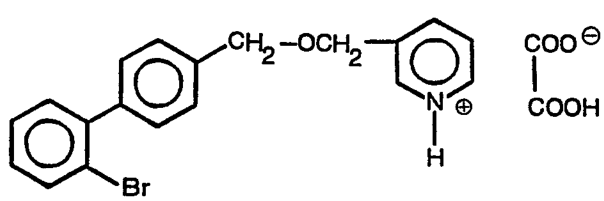

Experimentations

Divers essais toxicologiques et pharmacologiques ont été effectués sur les composés objet de l'invention.

A) Toxicologie

Les composés de la présente invention ont été soumis à des contrôles de toxicité. La toxicité des composés a été déterminée par la dose létale 50. Elle a été recherchée sur des lots de 10 souris par voie orale et intra-veineuse est calculée selon la méthode de MILLER et TAINTER (Proc. Soc. Exper. Biol. Med., 1944, *57*, 261). Les $DL_{50}$ des composés testés sont supérieures à 100 mg/kg par voie intra-veineuse et à 1000 mg/kg par voie orale.

B) Propriétés pharmacologiques

Les expérimentations pharmacologiques ont permis de mettre en évidence de remarquables propriétés hypolipémiante et hypocholestérolémiante. La vérification a été confirmée sur plusieurs tests.

Ci-après sont rapportés les résultats obtenus avec les produits des exemples 1, 6 et 7 qui ont été comparés au CLOFIBRATE et au FENOFIBRATE.

Test portant sur 4 jours de traitement voie orale selon la méthode de BUCHNAN, SPRANCMANIS et PARTYKA (J. Med. Chem., 12, 1001—1006, 1969). Rats males, naïfs, Sprague Dawley, répartis en lots homogènes de 8 animaux.

Traitement par voie orale par les produits à étudier en solution ou suspension dans la CMC 1% sous un volume de 10 ml/kg.

Durée de traitement: 4 jours

Fréquence des traitements: 1 fois par jour.

Le 5ème jour prélèvement de sang sur héparine à l'artère caudale après un jeûne de nourriture d'environ 16 heures.

Les résultats sont reportés dans le tableau suivant comparativement au CLOFIBRATE et au FENOFIBRATE.

| Produits | dosage mg/kg/j | cholestérol % baisse par rapport témoin | Consommation nourriture % variation par rapport témoin |
|---|---|---|---|
| CLOFIBRATE | 100 | —17 | —14 |
| | 200 | —8 (NS) | —10 |
| | 300 | —12 (NS) | —40 |
| FENOFIBRATE | 100 | —16 | —23 |
| | 200 | —27 | —10 |
| | 300 | —25 | —17 |
| EXEMPLE 1 | 25 | — 5(NS) | —57 |
| | 50 | —24 | —63 |
| F 2832 | 100 | —62 | —64 |
| EXEMPLE 6 | 25 | | —30 |
| | 50 | —27 | —41 |
| F 2876 | 100 | —75 | —76 |
| EXEMPLE 7 | 25 | —35 | —43 |
| | 50 | —51 | —52 |
| F 2875 | 100 | —77 | —46 |

C) Applications thérapeutiques

Compte tenu de leurs propriétés pharmacologiques, les composés de l'invention et plus

particulièrement les composés des exemples 1, 6 et 7 peuvent être utilisés en thérapeutique dans le traitement des différents types d'hyperlipidémies en vue de la prévention et du traitement de l'athérosclérose.

Les préparations pharmaceutiques contenant ces principes actifs peuvent être administrées par voie orale ou parentérale.

Il est possible d'y associer d'autres principes actifs pharmaceutiquement et thérapeutiquement acceptables.

**Revendications**

1. A titre de composés chimiques les dérivés d'alcools primaires halogéno biphényles répondant à la formule générale (I):

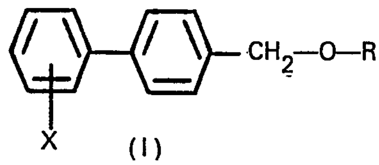

(I)

dans laquelle:

X représente un atome d'halogène en position ortho ou méta.

R représente un radical alcoyle, aminoalcoyle, pyridyl alcoyle, ou un radical acyle et plus particulièrement

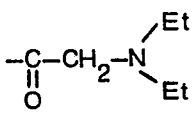 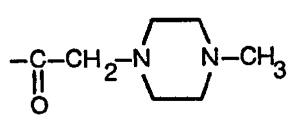, 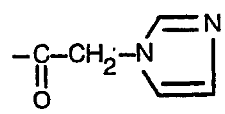 et 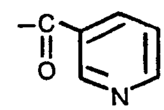, 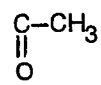

et dans le cas où R est salifiable, les sels thérapeutiquement acceptables avec des acides minéraux ou organiques.

2. Un composé selon la revendication 1, caractérisé en ce qu'il est choisi parmi:
hydrogénooxalate de (pyridyl-3 méthoxyméthyl)-4 chloro-2' biphényle (F 2832)
méthoxyméthyl)-4 chloro-2' biphényle (F 2816)
(éthoxyméthyl)-4 chloro-2' biphényle (F 2815)
(n-butoxyméthyl)-4 chloro-2' biphényle (F 2831)
hydrogénooxalate de [(diméthylamino-2 éthoxy) méthyl]-4 chloro-2' biphényle (F 2856)
dihydrogénomaléate de [(méthyl-4 pipérazinyl-1) acétoxyméthyl]-4 chloro-2' biphényle (F 2876)
hydrogénomaléate d'(imidazolyl) acétoxyméthyl)-4 chloro-2' biphényle (F 2875)
(pyridyl-3 carboxyméthyl)-4 chloro-2' biphényle (F 2882)
acétoxy méthyl-4 chloro-2' biphényle (F 2855)
hydrogénomaléate de chloro-2' (N,N-diéthylamino acétoxyméthyl)-4 biphényle (F 2870)
Chlorhydrate de (pyridyl-3 méthoxyméthyl)-4 fluoro-2' biphényle
Chlorhydrate de (pyridyl-3 méthoxyméthyl)-4 chloro-3' biphényle
(éthoxyméthyl)-4 chloro-3' biphényle
(éthoxyméthyl)-4 bromo-3' biphényle
Oxalate acide de (pyridyl-3 méthoxyméthyl)-4 bromo-2' biphényle

3. Procédé de préparation des composés chimiques selon les revendications 1 et 2 caractérisé en ce que l'on traite un alcool primaire halogéno biphényle de formule II:

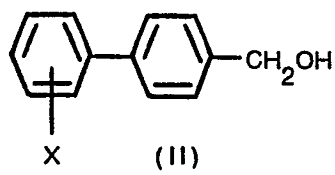

(II)

par un dérivé halogéné de formule: $Y—R_n$ pour obtenir les composés de formule générale (I):

X et R ayant les significations données à la revendication 1.

Y étant un halogène (n = 1) ou la fonction sulfate (n = 2).

4. A titre de médicaments nouveaux utiles notamment dans le traitement des troubles provoqués par l'athérosclérose les produits selon les revendications 1 et 2.

5. Compositions pharmaceutiques caractérisées en ce qu'elles contiennent comme principe actif au moins un composé selon l'une des revendications 1 et 2.

6. Compositions pharmaceutiques selon la revendication 5 caractérisées en ce qu'elles sont administrées par voie orale ou parentérale.

7. Compositions pharmaceutiques selon les revendications 5 et 6, caractérisées en ce qu'elles contiennent d'autres principes actifs associés aux composés chimiques des revendications 1 et 2.

**Patentansprüche**

1. Halogenbiphenol-Derivate von primären Alkoholen gemäß der allgemeinen Formel (I)

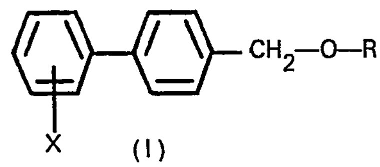

(I)

in welcher:

X ein Halogenatom in ortho- oder meta-Stellung bedeutet,

R ein Alkyl, Aminoalkyl, Pyridylalkyl, oder eine Acylgruppe bedeutet und im besonderen:

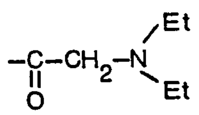 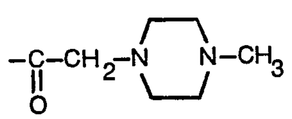, 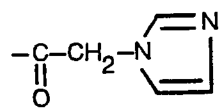 und 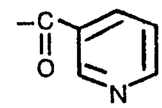, 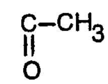

und in den Fällen, in welchen R fähig ist, Salze zu bilden, die therapeutisch verträglichen Salze von Mineral- oder organischen Säuren.

2. Verbindung nach Anspruch 1, dadurch gekennzeichnet, daß sie ausgewählt ist aus

Hydrogenoxalat von 4-(Pyridyl-3-methoxymethyl)-2'-chlorbiphenol (F 2832)
4-(Methoxymethyl)-2-chlor-biphenol (F 2816)
4-(Ethoxymethyl)-2'-chlor-biphenol (F 2815)
4-(n-Butoxymethyl)-2'-chlor-biphenol (F 2831)
Hydrogenoxalat von 4-[(Dimethylamino-ethoxy)-methyl]-2'-chlor-biphenol (F 2856)
Dihydrogenmaleat von 4-[(Methyl-4-piperazinyl-1)-acetoxymethyl]-2'-chlor-biphenol (F 2876)
Hydrogenmaleat von 4-(Imidazolyl-acetoxymethyl)-2'-chlorbiphenol (F 2875)
4-(Pyridyl-3-carboxymethyl)-2'-chlor-biphenol (F 2882)
4-Acetoxymethyl-2'-chlor-biphenol (F 2855)
— Hydrogenmaleat von 2'-Chlor-4-(N,N-diethylamino-acetoxymethyl)-biphenol (F 2870)
Chlorhydrat von 4-(Pyridyl-3-methoxymethyl)-2'-fluor-biphenol
Chlorhydrat von 4-(Pyridyl-3-methoxymethyl)-3'-chlor-biphenol
4-(Ethoxymethyl)-3'-chlor-biphenol
4-(Ethoxymethyl)-3'-brom-biphenol
saures Oxalat von 4-(Pyridyl-3-methoxymethyl)-2'-brom-biphenol.

3. Verfahren zur Herstellung der chemischen Verbindungen gemäß der Ansprüche 1 und 2, dadurch gekennzeichnet, daß man den primären Alkohol eines Halogenbiphenols der Formel II

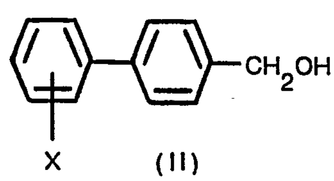

(II)

mit einer Halogenverbindung der Formel: $Y—R_n$ umsetzt und die Verbindungen der allgemeinen Formel (I) erhält,

wobei X und R die in Anspruch 1 definierten Bedeutungen aufweisen und

Y ein Halogen ($n = 1$) oder ein Sulfatrest ($n = 2$) ist.

4. Verbindungen der Ansprüche 1 und 2 als Medikament, im besonderen zur Behandlung von Beschwerden, die durch Arteriosklerose hervorgerufen werden.

5. Pharmazeutische Zusammensetzungen, dadurch gekennzeichnet, daß diese als aktiven Wirkstoff zumindest eine Verbindung der Ansprüche 1 und 2 enthalten.

6. Pharmazeutische Verbindungen nach Anspruch 5, dadurch gekennzeichnet, daß diese auf oralem oder parenteralem Wege verabreichbar sind.

7. Pharmazeutische Zusammensetzung nach den Ansprüchen 5 und 6, dadurch gekennzeichnet, daß diese andere aktive Wirkstoffe zusammen mit den chemischen Verbindungen der Ansprüche 1 und 2 enthalten.

**Claims**

1. As chemical compounds, the derivatives of primary halobiphenyl alcohols having the general formula (I):

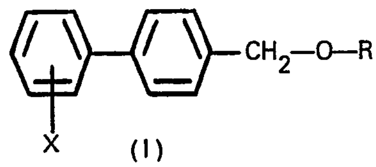

(I)

in which:

X represents a halogen atom in the ortho or meta position;

R represents an alkyl, aminoalkyl or pyridylalkyl radical or an acyl radical, especially:

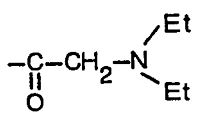
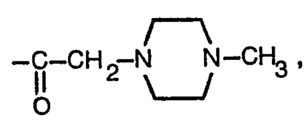,
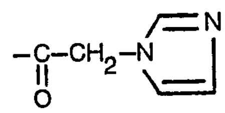
and
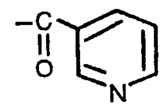,
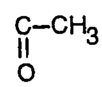

and, in the case where R is salt-forming, the therapeutically acceptable salts with mineral or organic acids.

2. A compound according to claim 1, characterised in that it is selected from:

4-(3-pyridylmethoxymethyl)-2′-chlorobiphenyl hydrogen oxalate (F 2832);
4-(methoxymethyl)-2′-chlorobiphenyl (F 2816);
4-(ethoxymethyl)-2′-chlorobiphenyl (F 2815);
4-(n-butoxymethyl)-2′-chlorobiphenyl (F 2831);
4-[(2-dimethylaminoethoxy)methyl]-2′-chlorobiphenyl hydrogen oxalate (F 2856);
4-[(4-methyl-1-piperazinyl)-acetoxymethyl]-2′-chlorobiphenyl di-hydrogen maleate (F 2876);
4-(imidazolylacetoxymethyl)-2′-chlorobiphenyl hydrogen maleate (F 2875);
4-(3-pyridylcarboxymethyl)-2′-chlorobiphenyl (F 2882);
4-acetoxymethyl-2′-chlorobiphenyl (F 2855);
2′-chloro-4-(N,N-diethylaminoacetoxymethyl)-biphenyl hydrogen maleate (F 2870);
4-(3-pyridylmethoxymethyl)-2′-fluorobiphenyl hydrochloride;
4-(3-pyridylmethoxymethyl)-3′-chlorobiphenyl hydrochloride;
4-(3-ethoxymethyl)-3′-chlorobiphenyl;
4-(ethoxymethyl)-3′-bromobiphenyl;
acid oxalate of 4-(3-pyridylmethoxymethyl)-2′-bromobiphenyl.

3. Process for the preparation of the chemical compounds according to claims 1 and 2, characterised in that a primary halobiphenyl alcohol of the formula II:

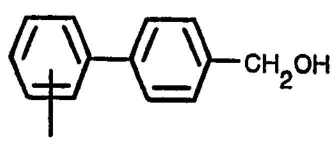

(II)

is treated with a halogenated derivative of the formula:

$Y—R_n$ to obtain the compounds of the general formula (I),

X and R having the meanings given in claim 1;

Y being a halogen (n = 1) or the sulphate function (n = 2).

4. The products according to claims 1 and 2 as novel medicaments for use especially in the treatment of disorders caused by arteriosclerosis.

5. Pharmaceutical compositions, characterised in that they contain as active ingredient at least one compound according to one of claims 1 and 2.

6. Pharmaceutical compositions according to claim 5, characterised in that they are administered orally or parenterally.

7. Pharmaceutical compositions according to claims 5 and 6, characterised in that they contain other active ingredients associated with the chemical compounds of claims 1 and 2.